# FASCICULE DE BREVET EUROPEEN

(11) **EP 2 890 438 B1**
(45) Date de publication et mention de la délivrance du brevet: **04.01.2017**
(21) Numéro de dépôt: 13766589.9
(22) Date de dépôt: 28.08.2013
(51) Int. Cl.: A61M 16/04, A61M 16/06, A61M 16/12, A61M 16/08, A61M 16/16

(54) **DISPOSITIF D'ASSISTANCE RESPIRATOIRE, APPAREIL NASAL ET MASQUE D'ASSISTANCE RESPIRATOIRE**
ATEMHILFEVORRICHTUNG, NASALE VORRICHTUNG UND ATEMHILFEMASKE
RESPIRATORY ASSISTANCE DEVICE, NASAL APPLIANCE AND RESPIRATORY ASSISTANCE MASK

(30) Priorité: 28.08.2012 FR 1258034
(43) Date de publication de la demande: 08.07.2015
(73) Titulaire: Boussignac, Georges, 92160 Antony (FR)
(72) Inventeur: Boussignac, Georges, 92160 Antony (FR)
(74) Mandataire: Jacobacci Coralis Harle
(86) Numéro de dépôt international: PCT/FR2013/051979
(87) Numéro de publication internationale: WO 2014/033401

(56) Documents cités:
- EP-A1- 0 245 142
- EP-A1- 0 390 684
- EP-A1- 0 911 051
- EP-A1- 2 228 088
- EP-A1- 2 239 004
- FR-A1- 2 827 778
- FR-A1- 2 911 073
- FR-A1- 2 942 967

## Description

La présente invention a pour objet un dispositif d'assistance respiratoire perfectionné, utilisable sur des patients dont la respiration spontanée est absente ou insuffisante, qu'ils soient placés ou non sous respiration artificielle. Elle est plus particulièrement adaptée aux dispositifs d'assistance respiratoire inventés par Monsieur Georges BOUSSIGNAC.

Le principe à la base du dispositif d'assistance respiratoire inventé par Monsieur BOUSSIGNAC consiste à injecter par un ensemble de canaux auxiliaires au moins un gaz respirable sous pression dans la lumière d'une tubulure reliée aux voies respiratoires du patient. Ce dispositif d'assistance a la particularité d'être du type ouvert, c'est-à-dire que la tubulure peut rester ouverte à l'air libre autorisant ainsi le passage de sondes ou autres accessoires de surveillance ou traitement du patient appelés dans la suite ancillaires. Plus précisément, le dispositif d'assistance est formé d'un élément tubulaire ou tube qui forme un canal principal et qui est destiné à être relié par son extrémité distale à une voie respiratoire d'un patient pour que ledit canal principal relie, à l'extérieur, le système respiratoire dudit patient. Le dispositif comporte en outre au moins un canal auxiliaire permettant l'injection d'un jet de gaz respirable destiné à la ventilation dudit patient et débouchant par un orifice de sortie distal dans ledit canal principal au voisinage de l'extrémité distale de ce dernier. Dans ce dispositif d'assistance, en regard de l'orifice de sortie distal de chaque canal auxiliaire, sont prévus des moyens de déflexion desdits jets de gaz respirable(s) vers l'intérieur dudit canal principal.

Ainsi, le jet de gaz respirable sous pression traversant ledit canal auxiliaire est défléchi vers l'axe du canal principal, lorsqu'il pénètre dans celui-ci. En aval desdits moyens de déflexion, c'est-à-dire à l'intérieur du canal principal, la pression dudit jet de gaz respirable chute et le jet sort à faible pression à travers l'extrémité distale de l'élément tubulaire. L'expérience a montré qu'en aval de la sortie distale de l'élément tubulaire, la pression est faible et maintenue constante dans tout l'espace respiratoire. De plus, cette pression est dépendante du débit de gaz respirable dans les canaux auxiliaires et on a donc également un moyen simple de réglage de la pression en jouant sur le débit du gaz dans les canaux auxiliaires.

Ce dispositif d'assistance dont on pourra compléter la connaissance dans la demande de brevet FR89/04280 a fait l'objet de divers perfectionnements dans les demandes de brevets suivantes données à titre d'exemple non exhaustif :
EP0390684, WO2008113913, EP2228088, FR2942967, WO2007118973, FR2911073, FR2813197, WO03039638, FR2827778, WO0010632, EP0978291 et EP0701834.

On verra d'ailleurs que ces divers perfectionnements peuvent être mis en oeuvre dans le cadre de la présente invention. Notamment la nébulisation de produits liquides, l'aide à la réanimation, l'utilisation nasale, le double flux, le freinage inspiratoire dans le cas d'un massage thoracique sur un patient en arrêt cardiorespiratoire...

Ce dispositif a apporté un confort certain aux patients et au personnel soignant du fait qu'il est du type ouvert. De plus, il est relativement simple dans son principe et à fabriquer.

Toutefois, le dispositif tel qu'il a été proposé présente l'inconvénient de générer un certain bruit du fait de l'injection de l'air qui crée des turbulences dans le dispositif et que ce dernier est ouvert.

Une solution simple serait l'ajout d'un filtre acoustique ou silencieux sur l'ouverture ou extrémité distale du dispositif, ledit filtre ou silencieux pouvant ou non permettre le passage des ancillaires et dans le dernier cas, devant être amovible pour être retiré lors des interventions pour passage desdits ancillaires. Cette solution simple présente cependant l'inconvénient d'ajouter de la complexité ou un accessoire au dispositif.

Or le demandeur s'est aperçu que, de façon surprenante, le fait de réduire le diamètre des orifices de sortie des jets et/ou celui des canaux auxiliaires de gaz respirable tout en augmentant le nombre des jets ou, d'une manière équivalente, de réunir les orifices de sortie des jets en une bande annulaire ou en des bandes semi-annulaires d'épaisseurs (= largeurs) réduites, chaque bande formant alors un orifice de sortie allongé propre, permettait de réduire de façon significative le bruit généré.

Plus précisément, le fait de passer d'un diamètre d'orifice de sortie de canal auxiliaire de l'ordre de 400 à 800 microns comme décrit dans la demande de brevet FR89/04280, à un diamètre inférieur à 150 microns et, encore mieux, compris entre 100 et 5 microns permet une réduction substantielle du bruit généré. Dans le cas de bandes, l'épaisseur de chaque bande correspondant à un orifice de sortie propre est alors inférieure à 150 microns et, encore mieux, comprise entre 100 et 5 microns.

Cette réduction de taille du/des orifices de sortie des jets, qu'ils soient ponctuels (ayant un diamètre réduit) ou en bandes (ayant une épaisseur réduite) outre la réduction du bruit présente l'avantage supplémentaire de réduire la consommation du gaz respirable des jets produits par les orifices de sortie, en général de l'oxygène. On a pu ainsi réduire de 50% la consommation en oxygène dans un dispositif à orifices de sortie de diamètre réduit avec des résultats acceptables par rapport à ceux produits dans des dispositifs classiques à diamètres d'orifices de sortie de jets habituels et à consommation d'oxygène habituelle. On peut expliquer ce phénomène par des modifications de la vitesse des jets et des conditions d'écoulement des gaz avec ces orifices de sortie plus petits.

Contrairement à ce que l'on pouvait craindre, cette réduction de diamètre ne présente pas de risque de bouchage/d'obstruction des orifices de sortie ou des canaux auxiliaires étant donné la haute qualité des gaz maintenant disponibles. Il est également possible d'installer un filtre à particules sur l'arrivée du gaz respirable en cas de crainte ou doute sur la propreté des gaz.

Il est à noter que des documents de l'état de la technique, notamment EP-2.228.088, FR-2.942.967, EP-2.239.004 et FR-2.980.978, mentionnent des canaux auxiliaires pour la création des jets, canaux dont il est écrit qu'ils ont un diamètre « de l'ordre de 5 à 800 microns ».

L'invention concerne donc un dispositif d'assistance respiratoire selon la revendication 1.

A noter que l'on mentionne la réunion des orifices de sortie car dans les moyens de production des jets de gaz respirable, au minimum les orifices de sortie distaux de jets sont fusionnés en bande(s) annulaire(s) ou semi-annulaire(s), et dans des variantes, les canaux auxiliaires sont également fusionnés formant des canaux auxiliaires en bande(s) annulaire(s) ou semi-annulaire(s).

Dans divers modes de mise en oeuvre de l'invention, les moyens suivants pouvant être utilisés seuls ou selon toutes les combinaisons techniquement possibles, sont employés :
- le diamètre de chaque orifice de sortie est inférieur ou égal à 100 microns,
- le diamètre de chaque orifice de sortie est compris entre 50 microns et 10 microns et est, de préférence d'environ 25 microns,
- le diamètre de chaque orifice de sortie est compris entre 20 microns et 5 microns et est, de préférence d'environ 10 microns,
- le diamètre de chaque orifice de sortie est compris entre 150 microns et 10 microns,
- le diamètre de chaque orifice de sortie est compris entre 150 microns et 100 microns,
- les canaux auxiliaires ont sur au moins une partie de leurs trajets un diamètre sensiblement égal à celui de leurs orifices de sortie,
- un canal auxiliaire donné débouche sur un seul orifice de sortie,
- un canal auxiliaire donné débouche sur plusieurs orifices de sortie,
- les orifices de sortie sont aux extrémités distales des canaux auxiliaires,
- les orifices de sortie sont décalés par rapport aux extrémités distales des canaux auxiliaires, lesdites extrémités distales des canaux auxiliaires au-delà des orifices de sortie étant en cul de sac,
- les diamètres de l'orifice de sortie et du canal auxiliaire correspondant sont identiques,
- au moins la/les parties distales du/des canaux auxiliaires débouchant dans le canal principal est/sont parallèles à celui-ci,
- le dispositif comporte un ensemble de canaux auxiliaires, lesdits canaux auxiliaires étant sensiblement parallèles à l'axe central dudit élément tubulaire sur au moins une partie de leurs trajets,
- le/les canaux auxiliaires sont ménagés dans la paroi de l'élément tubulaire,
- le dispositif comporte plusieurs canaux auxiliaires, chaque canal auxiliaire formant un passage tubulaire dans la paroi de l'élément tubulaire,
- le dispositif comporte un seul canal auxiliaire commun sur lequel les orifices de sortie sont reliés, ledit canal auxiliaire commun étant un passage annulaire ou une bague de distribution, externe et sensiblement coaxial et parallèle à l'axe central dudit élément tubulaire,
- dans le cas d'un seul canal auxiliaire formant un passage annulaire coaxial au canal principal, les orifices de sortie sont réunis ensembles en formant une sortie annulaire unique débouchant dans le canal principal, la sortie annulaire ayant une largeur/épaisseur correspondant au diamètre d'un orifice ponctuel individuel,
- certains des orifices de sortie adjacents entre eux sont réunis ensembles en formant une sortie unique en segment d'anneau (= semi-annulaire) débouchant dans le canal principal, la sortie en segment d'anneau ayant une largeur/épaisseur correspondant au diamètre d'un orifice ponctuel individuel,
- le dispositif comporte un orifice de sortie en bande annulaire,
- le dispositif comporte plusieurs orifices de sortie en bandes semi-annulaires,
- l'épaisseur de chaque orifice de sortie en bande annulaire ou semi-annulaire est comprise entre 50 microns et 10 microns et est, de préférence d'environ 25 microns,
- le/les orifices de sortie en bande(s) résultent de la réunion d'au moins certains des orifices de sortie distaux et de leurs canaux auxiliaires correspondants,
- le dispositif comporte en outre du/des orifices de sortie en bandes semi-annulaires des orifices de sortie ponctuels individualisés,
- le diamètre de chaque orifice ponctuel individualisé est compris entre 50 microns et 10 microns et est, de préférence d'environ 25 microns,
- dans le cas d'orifices de sortie réunis, les canaux auxiliaires correspondant sont également réunis en formant un canal auxiliaire annulaire ou semi-annulaire sur la totalité de leurs longueurs ou une partie seulement,
- l'orifice de sortie est formé dans une première face s'écartant dudit canal principal et les moyens de déflexion sont formés par une seconde face, inclinée, dudit canal principal, disposée en regard de ladite première face et convergente en direction de l'orifice de sortie,
- la portion terminale du canal principal côté extrémité distale est évasée,
- la seconde face est prolongée vers l'extrémité distale du canal principal par une paroi évasant légèrement ledit canal principal,
- les moyens de déflexion sont formés directement dans la paroi interne de l'élément tubulaire,
- les moyens de déflexion sont formés sur un embout rapporté à l'extrémité distale de l'élément tubulaire,
- les moyens de déflexion consistent en un ensemble discontinu d'évidements de forme générale conique, ménagés dans la paroi interne, et au fond de chacun desquels débouche l'extrémité distale d'un canal auxiliaire par son orifice de sortie,
- le dispositif comporte une pluralité de canaux auxiliaires dont au moins certains sont alimentés en commun en gaz respirable sous pression,
- les canaux auxiliaires non alimentés en commun, servent à l'introduction de produits gazeux additionnels, tels que des produits médicamenteux ou des gaz humides,
- au moins une partie des canaux auxiliaires sont alimentés en commun en gaz respirable par l'intermédiaire d'une bague de distribution, coaxiale à l'élément tubulaire,
- au moins une autre partie des canaux auxiliaires sont alimentés en commun par des produits médicamenteux ou de l'humidité,
- la portion terminale du canal principal côté extrémité distale comporte une partie rétrécie,
- la portion terminale du canal principal côté extrémité distale comporte une bague de rétrécissement,
- les orifices de sortie de canaux auxiliaires sont disposés en couronne,
- le dispositif comporte au moins une couronne d'orifices de sortie de canaux auxiliaires, les orifices de sortie d'une couronne étant disposés le long d'une section transversale dudit élément tubulaire,
- le dispositif comporte au moins deux couronnes d'orifices de sortie de canaux auxiliaires décalés le long dudit élément tubulaire, les orifices de sortie de couronnes différentes provenant de canaux auxiliaires identiques, un même canal auxiliaire pouvant déboucher sur plusieurs orifices de sortie,
- le dispositif comporte au moins deux couronnes d'orifices de sortie de canaux auxiliaires décalés le long dudit élément tubulaire, les orifices de sortie de couronnes différentes provenant de canaux auxiliaires différents, un canal auxiliaire donné ne débouchant que sur un seul orifice de sortie,
- les orifices de sortie d'une couronne ou une partie de ceux-ci sont réunis pour former un orifice commun annulaire ou semi annulaire,
- le dispositif comporte au moins deux couronnes d'orifices de sortie en bandes annulaires ou semi annulaires,
- le dispositif à couronnes d'orifices comporte en outre de son/ses orifices de sortie en bandes annulaires et/ou semi-annulaires des orifices de sortie ponctuels,
- les moyens de déflexion permettent de faire converger les uns vers les autres, à l'intérieur du canal principal, les jets de gaz respirable(s) des canaux auxiliaires,
- les moyens de déflexion sont configurés de manière à permettre d'orienter les jets vers l'extrémité distale de l'élément tubulaire selon une incidence, par rapport à l'axe central dudit élément tubulaire, comprise entre 90°, l'axe des jets étant perpendiculaire audit axe central, et 25°, l'axe des jets croisant l'axe central selon un angle de 25°,
- tous les jets ont la même incidence,
- les jets ont des incidences différentes selon les orifices de sortie,
- les jets ont des incidences différentes selon la couronne d'orifices de sortie,
- l'incidence des jets est d'environ 45°,
et/ou, dans certaines variante
- le dispositif est à double flux inversés permettant de favoriser l'expiration aussi bien que l'inspiration d'un patient, le dispositif comportant au moins un canal auxiliaire supplémentaire, indépendant du/des premiers canaux auxiliaires des jets de l'extrémité distale du canal principal, et relié à une source de gaz sous pression, ledit au moins un canal auxiliaire supplémentaire débouchant dans le canal principal au voisinage de l'extrémité proximale de ce dernier, tandis que, en regard de chaque orifice de sortie du canal auxiliaire supplémentaire correspondant, sont prévus des moyens pour la déflexion du jet de gaz traversant ce dernier en direction de l'intérieur dudit canal principal,
- au moins l'/les extrémités dudit/desdits canaux auxiliaires supplémentaires débouchant par des orifices de sortie respectifs dans le canal principal est/sont parallèle à celui-ci,
- de préférence, dans le cas d'un dispositif double flux avec canal auxiliaire supplémentaire, la partie proximale du dispositif est semblable sinon identique, symétriquement, à la partie distale dudit dispositif, au moins en ce qui concerne la disposition desdits canaux auxiliaires et lesdits moyens de déflexion,
- le dispositif constitue l'embout d'entrée et de sortie d'air d'un masque d'assistance respiratoire destiné à être appliqué sur le visage d'un patient,
- le dispositif constituant l'embout d'entrée et de sortie d'air d'un masque d'assistance respiratoire est amovible,
et/ou, dans certaines variantes :
- le dispositif comporte en outre des moyens de dérivation aptes à dériver une fraction de volume dudit gaz respirable destiné audit canal auxiliaire avant son entrée dans ce dernier et des moyens d'aspiration d'air ambiant entraînés par ladite fraction dérivée de gaz respirable et lesdits moyens d'aspiration sont reliés au canal principal de sorte que les moyens d'aspiration sont aptes à acheminer, dans ledit canal principal l'air ambiant aspiré mélangé à ladite fraction dérivée de gaz respirable,
- le dispositif comporte des moyens de réglage de la fraction de gaz respirable dérivée par lesdits moyens de dérivation,
- les moyens de réglage de la fraction de gaz respirable dérivée sont disposés entre lesdits moyens de dérivation et les moyens d'aspiration d'air ambiant,
- les moyens de réglage de la fraction de gaz respirable dérivée comportent au moins une vanne,
- le dispositif comporte en outre des moyens de régulation de débit de gaz respirable dilué sortant desdits moyens d'aspiration et destiné à entrer dans ledit canal principal,
- les moyens de régulation de débit de gaz respirable dilué sont agencés entre lesdits moyens d'aspiration d'air ambiant et des moyens de communication fluidique,
- les moyens de régulation de débit de gaz respirable dilué comportent au moins une vanne,
- le débit de gaz respirable dilué sortant desdits moyens d'aspiration passe dans le canal principal par un orifice de communication qui est ménagé dans la paroi dudit dispositif,
- le canal principal est formé par un tube,
- le canal principal est formé par un tube souple,
- le débit de gaz respirable dilué sortant desdits moyens d'aspiration passe par une gaine souple étanche qui enveloppe, au moins sur une partie de sa longueur ledit tube souple formant le canal principal et qui forme une voie périphérique autour dudit tube souple et dans laquelle débouche l'orifice de communication,
- les moyens d'aspiration sont montés directement sur ledit tube souple au voisinage de son extrémité proximale,
- l'orifice de communication est disposé entre les moyens de déflexion des jets et l'extrémité distale du canal principal,
- l'orifice de communication est divisé en des orifices de communication multiples,
et/ou, dans certaines variantes :
- le dispositif comporte en outre des moyens de freinage spontané de l'entrée d'air extérieur dans le canal principal via son extrémité proximale,
- les moyens de freinage de l'entrée d'air extérieur dans le canal principal comportent un corps creux pourvu d'une première et d'une seconde soupapes normalement fermées, la première soupape étant apte à s'ouvrir spontanément et immédiatement lors d'une surpression/contrepression provenant de la voie respiratoire du patient, notamment lors d'une compression thoracique, alors que la seconde soupape est apte à s'ouvrir spontanément, mais progressivement, en dehors de ladite surpression/contrepression, notamment lors de la suppression d'une compression thoracique, et le corps creux est disposé à l'extrémité proximale de l'élément tubulaire du dispositif,
- les première et seconde soupapes sont disposées en parallèle entre l'extérieur et la cavité interne du corps creux,
- les première et seconde soupapes sont disposées en série entre l'extérieur et la cavité interne du corps creux, l'une desdites soupapes étant portée par l'autre,
- la première soupape est constituée par une membrane élastique s'appliquant spontanément contre un siège prévu dans ledit corps creux et est liée audit siège par des points de fixation répartis à sa périphérie, l'air chassé lors des surpression/contrepression passant librement de la cavité du corps creux à l'extérieur par des passages qui se forment spontanément et immédiatement par la déformation élastique de ladite membrane entre lesdits points de fixation et ledit siège et la seconde soupape est formée par au moins une fente à bords jointifs pratiquée dans ladite membrane, l'air aspiré lors de la suppression de la surpression/contrepression passant progressivement en étant freiné de l'extérieur à la cavité du corps creux par le passage qui se forme spontanément dans ladite membrane par déformation élastique de celle-ci entraînant l'écartement progressif de ses bords jointifs,
- les moyens de freinage de l'entrée d'air extérieur dans l'élément tubulaire du dispositif font partie intégrante de celui-ci,
- les moyens de freinage de l'entrée d'air extérieur dans l'élément tubulaire du dispositif sont rapportés de façon amovible à celui-ci,
et/ou, dans certaines variantes :
- l'élément tubulaire formant le canal principal du dispositif comporte au moins un orifice latéral de sécurité qui traverse sa paroi latérale au moins sensiblement en regard du point de convergence des jets de gaz respirable(s) et qui est apte à relier à l'extérieur la partie dudit canal principal qui se trouve côté distal/en aval par rapport au sens des jets de gaz respirable(s) et par rapport aux moyens de déflexion,
- la partie distale du canal principal est en communication directe avec l'extérieur à travers ledit orifice latéral de sécurité,
- l'orifice latéral de sécurité est fermé par un bouchon amovible,
- l'orifice latéral de sécurité est fermé par un bouchon amovible imperdable,
- le dispositif comporte des moyens fibreux ou poreux pour masquer le bruit des jets de gaz respiratoire traversant ledit orifice latéral de sécurité,
- le dispositif comporte un conduit reliant l'orifice latéral de sécurité à l'extérieur,
- les moyens fibreux ou poreux sont dans le conduit,
- le conduit est constitué par un conduit coaxial entourant l'élément tubulaire,
- le conduit coaxial débouche à l'extérieur du côté de l'extrémité proximale dudit élément tubulaire,
- le conduit coaxial débouche à l'extérieur du côté de l'extrémité distale dudit élément tubulaire,
- le conduit est constitué par une gaine souple entourant l'élément tubulaire et débouchant à l'extérieur du côté de l'extrémité proximale dudit élément tubulaire,
- le conduit est constitué par une gaine souple entourant l'élément tubulaire et débouchant à l'extérieur du côté de l'extrémité distale dudit élément tubulaire,
- la partie proximale du canal principal comporte des obstacles internes saillants empêchant l'obturation hermétique de l'extrémité proximale,
et/ou, dans certaines variantes :
- afin que le dispositif puisse délivrer à la voie respiratoire du patient un débit prédéterminé de gaz respiratoire(s) sous une pression d'utilisation dont la valeur doit être comprise dans une plage de valeurs d'utilisation pour assurer l'efficacité dudit dispositif sans mettre en danger ledit patient, ledit dispositif étant relié à la source de gaz respiratoire(s) par un conduit d'alimentation de gaz et à une pression d'alimentation comprise entre une valeur minimale et une valeur maximale, le conduit d'alimentation en gaz respiratoire(s) comporte un élément de perte de charge spécifique assurant que ladite pression d'utilisation est au plus égale à la valeur supérieure de ladite plage de valeurs d'utilisation, lorsque ladite pression d'alimentation est à ladite valeur maximale,
- l'élément de perte de charge spécifique est constitué par un bouchon apte à obturer le conduit d'alimentation en gaz respiratoire et percé d'un passage longitudinal pour le/les gaz,
- la valeur de la perte de charge apportée par ledit élément de perte de charge spécifique est ajustée par la longueur du bouchon percé,
- l'élément de perte de charge spécifique est constitué par un tronçon d'un profilé,
et/ou, dans certaines variantes :
- le dispositif comporte entre les moyens de déflexion et l'extrémité distale du canal principal, des moyens de communication commandables à l'ouverture et à la fermeture et aptes, lorsqu'ils sont en position ouverte, à former un passage reliant ledit canal principal à l'environnement extérieur,
- le passage reliant le canal principal à l'environnement extérieur a une section variable,
- les moyens de communication sont du type à bague tournante percée latéralement et apte à découvrir des passages de diamètres différents,
- la bague tournante est montée directement sur l'élément tubulaire,
- la bague tournante est montée sur une cheminée en communication avec le canal principal,
et/ou, dans certaines variantes :
- le dispositif comporte en outre au moins un conduit d'apport alimenté en produit liquide à nébuliser dans le canal principal et des moyens de nébulisation,
- le dispositif comporte en outre au moins un conduit d'apport rapporté alimenté en produit liquide à nébuliser et logé dans le canal principal, ledit conduit d'apport pénétrant dans le canal principal à travers l'extrémité proximale de l'élément tubulaire et débouchant dans ledit canal principal au voisinage du débouché, dans ce dernier, du/des orifices de sortie du/des canaux auxiliaires,
- le diamètre interne dudit conduit d'apport rapporté est de l'ordre de 200 à 300 microns,
- à l'extérieur de l'élément tubulaire, le conduit d'apport rapporté est ascendant et au moins approximativement perpendiculaire à l'axe de l'élément tubulaire,
- le conduit d'apport est rapporté à l'élément tubulaire de façon amovible,
- le conduit d'apport est en outre fixé à l'élément tubulaire au moyen d'une pince disposée à cheval sur le bord de l'extrémité proximale de l'élément tubulaire,
- l'extrémité côté distal du conduit d'apport rapporté présente la forme d'un biseau,
- l'extrémité côté distal du conduit d'apport rapporté se trouve au voisinage des moyens de déflexion,
- le conduit d'apport rapporté comporte une pluralité d'orifices d'apports distaux indépendants,
- le conduit d'apport rapporté comporte une pluralité de canaux indépendants,
et/ou, dans certaines variantes :
- un conduit d'alimentation de gaz relie le/les canaux auxiliaires du dispositif à/aux sources de gaz respirables, ledit conduit d'alimentation de gaz comportant du côté de la/des sources, un dispositif à perte de charge apte à limiter le débit et la pression dudit gaz respirable disponible à la sortie de ladite source et à imposer audit jet de gaz respirable une valeur de débit et une valeur de pression prédéterminées et, du côté du/des canaux auxiliaires, comporte une soupape d'échappement tarée apte à mettre ledit conduit d'alimentation de gaz en communication avec l'atmosphère dès que la pression dans ledit conduit d'alimentation de gaz dépasse ladite valeur de pression prédéterminée,
- le dispositif à perte de charge est réglable de façon à permettre d'imposer aux jets de gaz respirable une pluralité de valeurs de débit et de valeurs de pression prédéterminées ou préréglées,
- le tarage de la soupape d'échappement tarée est réglable,
- le dispositif à perte de charge est incorporé à l'élément tubulaire,
- le dispositif à perte de charge est extérieur à l'élément tubulaire,
- la soupape d'échappement tarée est incorporée à l'élément tubulaire,
- la soupape d'échappement tarée est extérieure à l'élément tubulaire,
- le dispositif comporte un humidificateur dans la conduite d'alimentation de gaz reliant la/les sources de gaz respirables à/aux canaux auxiliaires,
- l'humidificateur est disposé entre le dispositif à perte de charge et la soupape d'échappement tarée,
et/ou, dans certaines variantes :
- le dispositif comporte une vanne commandée susceptible d'obturer au moins partiellement l'extrémité proximale du canal principal au moins pendant l'insufflation du gaz respirable,
- la vanne commandée forme un ensemble monolithique avec l'élément tubulaire,
- la vanne commandée est rapportée sur l'élément tubulaire,
- la vanne commandée est solidaire d'un embout susceptible d'être emboîté sur l'extrémité proximale de l'élément tubulaire,
- la vanne commandée comporte une enceinte étanche à section toroïdale, disposée au voisinage de l'extrémité proximale du canal principal et comportant au moins une paroi interne souple et élastique qui, en se dilatant ou en se rétractant en réponse à l'introduction ou à l'évacuation d'un fluide de gonflage dans ladite enceinte étanche, commande la section de passage dudit canal principal,
- le bord libre de l'extrémité proximale de l'élément tubulaire comporte au moins une échancrure,
- le dispositif comporte au moins une prise de pression disposée du côté de l'extrémité distale de l'élément tubulaire,
- la prise de pression comporte une chambre périphérique annulaire coaxiale à l'élément tubulaire et débouchant côté distal du dispositif par un passage annulaire distal, ladite chambre périphérique annulaire étant en relation avec un embout de sortie latéral,
- un filtre, fibreux ou poreux annulaire est disposé dans la chambre périphérique annulaire,
- la prise de pression est formée par l'orifice de sortie par lequel un des canaux auxiliaires ménagés dans la paroi de l'élément tubulaire débouche au voisinage de l'extrémité distale dudit élément tubulaire,
- le dispositif comporte des moyens recevant la pression prélevée par ladite prise de pression et susceptibles d'imposer l'ouverture à la vanne commandée pour dégager le canal principal,
- le dispositif comporte une vanne d'échappement tarée disposée à l'extrémité proximale de l'élément tubulaire, du côté opposé au bord libre de l'extrémité proximale par rapport à ladite vanne commandée,
- l'introduction et l'évacuation du fluide de gonflage dans/de ladite enceinte étanche résulte du déplacement bidirectionnel d'un volume de fluide approprié contenu dans une capacité tampon à volume variable commandable,
- le fluide de gonflage est un gaz,
et/ou dans une application particulière :
- le dispositif est spécialement configuré pour être mis en oeuvre sur un patient en arrêt cardiorespiratoire et sur lequel un massage thoracique est effectué avec des phases de compression thoracique pour expiration et de relâchement thoracique (= décompression thoracique) pour inspiration et le dispositif comporte en outre dans son canal principal au moins un moyen de freinage proximal de la circulation d'air et/ou d'oxygène inspiré par l'extrémité proximale en début d'inspiration afin d'augmenter la dépression intrathoracique au début de l'inspiration, ledit moyen de freinage étant soit un moyen passif, soit un moyen actif, le moyen passif étant choisi parmi une/des soupapes à membrane souple et/ou une/des soupapes à clapets, le moyen actif comportant un équipement de mesure de pression distale, un équipement de commande et un équipement commandé de réduction de passage d'une partie proximale du canal principal, l'équipement de commande étant configuré pour commander la réduction de passage par l'équipement commandé seulement lors du début d'une inspiration détectée par des mesures de l'équipement de mesure de pression distale,
- le moyen de freinage proximal est intégré au dispositif de l'invention,
- le moyen de freinage proximal est rapporté sur l'extrémité proximale du dispositif de l'invention,
- le moyen de freinage proximal passif comporte deux soupapes à clapet et ressort, les dites deux soupapes étant en parallèle ou en série,
- le moyen de freinage proximal passif comporte une soupape à membrane souple,
- le moyen de freinage proximal actif comporte un équipement commandé de réduction de passage à boudins gonflables,
- l'équipement de mesure de pression distale est remplacé par un équipement de mesure thoracique des compressions de relâchements du massage thoracique.

L'invention concerne également un appareil nasal d'assistance respiratoire pour un patient comportant un dispositif selon l'invention pour chaque narine du patient, les deux dispositifs étant destinés à être alimentés en gaz respirable(s) en parallèle, chaque canal principal d'un dispositif étant destiné à être relié par son extrémité distale au système respiratoire du patient par l'intermédiaire d'une de ses narines.

Dans divers modes de mise en oeuvre de l'appareil, les moyens suivants pouvant être utilisés seuls ou selon toutes les combinaisons techniquement possibles, sont employés :
- le dispositif incorporé à l'appareil présente une ou plusieurs des caractéristiques décrites concernant ledit dispositif,
- l'appareil comporte une pièce monolithique incorporant deux dispositifs selon l'invention,
- dans la pièce monolithique les deux éléments tubulaires sont parallèles entres eux,
- l'appareil comporte en outre un moyen d'apport de liquide à nébuliser dans le flux gazeux traversant l'appareil vers le patient,
- l'appareil comporte en relation avec chaque élément tubulaire deux parties de diamètres différents disposées en série, la partie de plus grand diamètre étant proximale, c'est-à-dire du côté de l'air libre opposé au côté du patient, et étant pourvue d'un raccord tubulaire latéral,
- les parties de plus grand et de plus petit diamètres sont séparées par une partie intermédiaire conique,
- l'appareil comporte, pour chaque partie de plus grand diamètre un manchon disposé dans ladite partie de plus grand diamètre et y ménageant un espace de section annulaire, le raccord tubulaire latéral débouchant dans ledit espace de section annulaire, des moyens de solidarisation du manchon à ladite partie de plus grand diamètre obturant ledit espace de section annulaire du côté proximal opposé à une partie intermédiaire conique reliant la partie de plus grand diamètre proximale de la partie de plus petit diamètre,
- l'appareil comporte pour chaque partie intermédiaire conique un conduit latéral d'apport de liquide débouchant dans ladite partie intermédiaire conique, au voisinage du raccord de celle-ci et de la partie de plus petit diamètre,
- chaque conduit latéral d'apport fait saillie à l'intérieur de ladite partie intermédiaire conique et présente un biseau dont l'inclinaison est opposée à celle de ladite partie intermédiaire conique, à l'endroit où débouche ledit conduit latéral,
- l'appareil est pourvu d'un dispositif d'alimentation en gaz respirable(s), associé à une source d'un/de tel(s) gaz et pourvu d'un détecteur de pression apte à détecter le début des expirations et le début des inspirations du patient ventilé, ledit dispositif d'alimentation comportant en outre, alimentés en parallèle par ladite source, des moyens de calibrage délivrant en permanence un courant de gaz respirable(s) sous une première pression apte à maintenir ouvertes les bronchioles dudit patient en évitant le collapsus et une vanne commandable calibrée pour délivrer, lorsqu'elle est ouverte, un courant de gaz respirable(s) sous une seconde pression, supérieure à ladite première pression et apte à assister efficacement l'inspiration dudit patient, ladite vanne commandable étant commandée par ledit détecteur de pression, à la fermeture pendant les expirations et à l'ouverture pendant les inspirations,
- le dispositif d'alimentation comporte un débitmètre interposé entre les moyens de calibrage et la vanne commandable, d'une part, et les canaux auxiliaires, d'autre part,
- le dispositif d'alimentation comporte un dispositif d'alarme commandé par le détecteur de pression,
- une prise de pression pour le détecteur débouche dans au moins l'un des éléments tubulaires en aval, côté distal, c'est-à-dire côté patient, desdits moyens de déflexion et au voisinage de l'extrémité distale dudit élément tubulaire, ladite prise de pression étant reliée au détecteur par un tube de raccordement,
- la prise de pression est un trou traversant la paroi de l'élément tubulaire et le tube de raccordement est extérieur à ce dernier,
- le tube de raccordement est disposé à l'intérieur de l'élément tubulaire et la prise de pression est formée par une échancrure latérale, pratiquée dans le tube de raccordement et ayant une longueur égale à plusieurs fois le diamètre dudit tube de raccordement,
- l'appareil comporte des organes mobiles aptes à restreindre la section de passage des jets gazeux au niveau des moyens de déflexion,
- les organes mobiles sont constitués par les extrémités distales de fourreaux pouvant glisser à frottement dur à l'intérieur des canaux principaux,
- l'appareil comporte, sur chacune de ses deux extrémités distales, un manchon de narine, souple, rapporté extérieurement, et destiné à venir au contact de la paroi interne de la narine correspondante,
- chaque manchon de narine comporte une ouverture en regard de l'ouverture distale de l'élément tubulaire,
- chaque manchon de narine est en une matière mousse à cellules ouvertes et obture l'ouverture distale de l'extrémité distale de l'élément tubulaire, tout en assurant le passage aux gaz respirable(s) et aux éventuels liquides nébulisés.

L'invention concerne également un masque d'assistance respiratoire muni d'un dispositif selon l'invention. Le dispositif en relation avec le masque présente une ou plusieurs des caractéristiques décrites concernant ledit dispositif.

La présente invention va maintenant être exemplifiée sans pour autant en être limitée avec la description qui suit en relation avec les figures suivantes:
- la Figure 1 est une vue schématique et partielle, en coupe axiale agrandie, d'un premier mode de réalisation du dispositif de l'invention,
- les Figures 2 et 3 sont des coupes transversales, respectivement selon les lignes II-II et III-III de la figure 1,
- les Figures 4 et 5 illustrent, schématiquement en coupe axiale agrandie, deux variantes de réalisation pour l'extrémité distale du dispositif selon l'invention,
- la Figure 6 montre, en coupe axiale schématique et partielle, une variante à double flux opposés du dispositif selon la présente invention,
- la Figure 7 montre, en coupe axiale, une autre variante de réalisation du dispositif de l'invention, où l'élément tubulaire n'est plus destiné à être introduit directement dans une voie respiratoire, mais constitue l'embout d'un masque d'assistance respiratoire destiné à être appliqué sur le visage (bouche et/ou nez) d'un patient.
- la Figure 8 montre une application de l'invention sous forme d'un appareil nasal d'assistance respiratoire pour un patient,
- la figure 9 montre, en coupe axiale, une variante de réalisation du dispositif de l'invention,
- les figures 10, 11 et 12 sont des coupes transversales du dispositif de la figure 9, respectivement selon les lignes III-III, IV-IV et V-V, et
- la Figure 13 montre une vue schématique, partiellement en coupe axiale, d'un masque respiratoire équipé d'une variante de réalisation du dispositif de l'invention.

Le dispositif conforme à l'invention permet l'intubation sans arrêt de l'assistance respiratoire, l'injection par voie respiratoire de médicaments, d'anesthésiques ou d'humidité pendant l'assistance respiratoire, la mesure dynamique des pressions, l'augmentation du volume échangé car la pression est autolimitée et il n'y a aucun risque d'écrasement des capillaires pulmonaires. Il permet la diminution pour une même quantité d'oxygène échangée de l'importance d'oxygène dans le mélange, ce qui diminue d'autant les effets secondaires de l'assistance et la possibilité d'utiliser des respirateurs moins coûteux que les respirateurs précédemment utilisés.

Le dispositif d'assistance respiratoire selon l'invention permet d'utiliser le différentiel de pression minimum nécessaire en apportant un volume de gaz respirable maximum. Par ailleurs, la configuration physique du dispositif de l'invention, notamment le canal principal, permet de conserver un diamètre intérieur suffisant pour permettre le passage des instruments et des sondes médicales que l'opérateur veut utiliser.

Dans son principe, le dispositif de l'invention est donc constitué d'un élément tubulaire, en général un tube, qui forme un canal principal et qui est destiné à être relié par son extrémité distale à la voie respiratoire du patient alors que l'extrémité proximale de l'élément tubulaire débouche à l'air libre à l'extérieur du patient, le système respiratoire du patient étant donc relié à l'extérieur par l'intermédiaire du canal principal du dispositif. Des canaux auxiliaires sont pratiqués dans l'épaisseur de l'élément tubulaire et débouchent dans le canal principal. Les canaux auxiliaires sont alimentés en gaz respiratoire(s) par l'intermédiaire d'un conduit d'alimentation. Le dispositif comporte des moyens de déflexion pour faire converger les uns vers les autres, à l'intérieur du canal principal, les jets de gaz respiratoire(s) injectés par lesdits canaux auxiliaires vers le canal principal. De préférence, les moyens de déflexion des jets de gaz respirable(s) dévient les jets vers l'axe central du canal principal.

Toujours dans son principe, le dispositif de l'invention comporte au moins un orifice de sortie en forme de bande annulaire ou semi-annulaire résultant de la réunion d'au moins certains orifices de sortie distaux, l'épaisseur de l'orifice de sortie en forme de bande étant inférieure à 150 microns. Dans certains cas, des orifices de sortie ponctuels sont en outre présents et lesdits orifices ponctuels ont un diamètre inférieur à 150 microns.

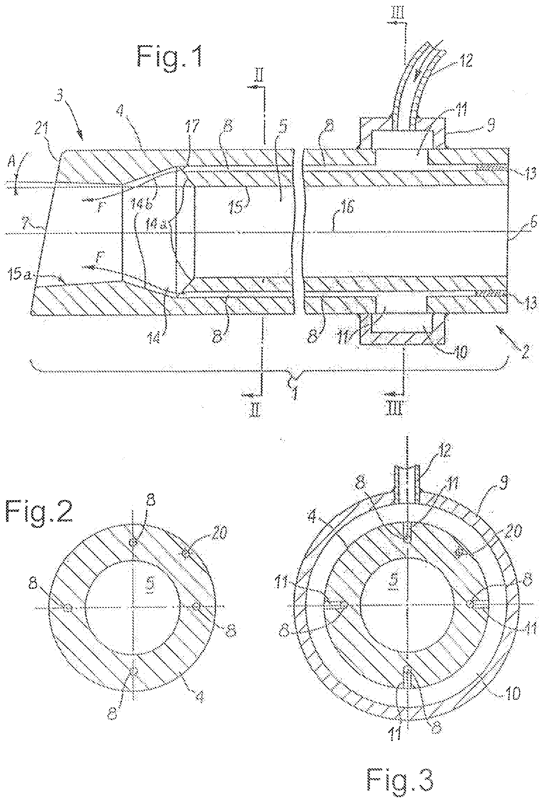

Sur la Figure 1, on a représenté schématiquement et à grande échelle, l'élément tubulaire avec ses extrémités proximale 2 et distale 3 d'un mode de réalisation 1 du dispositif selon l'invention. Ce mode de réalisation peut constituer ou être combiné à, par exemple, une sonde endotrachéale, oro-nasale avec ou sans ballonnet, une sonde endotrachéale pédiatrique, une sonde de monitorage des gaz, une sonde endobronchique, une sonde nasopharyngée, une sonde d'intubation anatomique pour enfant, une sonde de Cole néonatale, une sonde canule de Guedel, ou une sonde nasale d'oxygénothérapie.

Le dispositif 1 comporte un élément tubulaire 4, souple ou préformé, notamment pour s'adapter à la morphologie du patient, délimitant un canal principal 5 débouchant, par l'orifice proximal 6, à l'extrémité proximale 2, et, par l'orifice distal 7, à l'extrémité distale 3.

Ainsi, le canal principal 5 est capable d'assurer le passage entre les orifices 6 et 7, dont l'un, l'orifice distal 7, est destiné à se trouver à l'intérieur des voies respiratoires d'un patient ou à l'extérieur en relation avec ces dernières et l'autre, l'orifice proximal 6, est destiné à se trouver en communication avec l'environnement extérieur dudit patient. Cet orifice proximal 6 peut déboucher à l'air libre et, dans ce cas, le patient peut inspirer de l'air frais et expirer l'air vicié à travers le canal principal 5. On peut également relier l'orifice proximal 6 à au moins une source de gaz respirable(s) sous pression (non représentée) et prévoir un système de valves unidirectionnelles, pour que le patient inspire le(les) gaz respirable de ladite source à travers ledit canal principal 5 et expire le gaz vicié à l'air libre, également à travers ce canal principal 5.

Le diamètre du canal principal 5 est de l'ordre de quelques millimètres. Des essais satisfaisants ont été effectués avec des diamètres de 3 mm, 7 mm et 8 mm, voir un peu plus.

Par ailleurs, dans l'épaisseur de la paroi de l'élément tubulaire 4 sont ménagés des canaux auxiliaires 8, s'étendant sur la presque totalité de la longueur du canal principal 5. Ces canaux auxiliaires 8 sont destinés à être reliés à une (ou plusieurs) source de gaz respirable sous pression (non représentée). Par exemple, cette pression est de quelques bars, par exemple 1,2 ou 4 bars, et elle est réglable. De préférence, comme représentés, ces canaux auxiliaires sont des canaux individualisés sur au moins une partie de leurs trajets. Dans des variantes, les canaux auxiliaires ont une partie commune formant une chambre circulaire coaxiale et extérieure au canal principal ou sont réduits pour ce qui concerne leur trajet individuel aux orifices de sortie débouchant dans le canal principal et, dans ce dernier cas, on peut considérer qu'il n'y a qu'un canal auxiliaire. Dans tous les cas, le/les canaux auxiliaires débouchent dans le canal principal par plusieurs orifices de sortie pour y former des jets de gaz respirable(s).

Comme cela est représenté sur les Figures 1 et 3, la liaison à la ou aux sources de gaz respirables sous pression peut être réalisée au moyen d'une bague 9, entourant de façon étanche l'élément tubulaire 4, du côté de l'extrémité proximale 2, et délimitant une chambre annulaire étanche 10 autour dudit élément tubulaire. Les canaux auxiliaires 8 sont mis en communication avec la chambre 10 grâce à des arrachements locaux 11 de la paroi de l'élément tubulaire 4 et ladite chambre 10 est reliée à/aux sources de gaz respirable(s) par une liaison 12. Bien entendu, les extrémités proximales des canaux auxiliaires 8 sont obturées, par exemple par des bouchons 13.

Les canaux auxiliaires 8 ont un diamètre plus petit que celui du canal principal 5. Le diamètre des canaux auxiliaires 8 est de préférence voisin ou inférieur à 150 microns afin de réduire/supprimer les bruits générés par les jets de gaz dans le canal principal.

Du côté distal, chacun des canaux auxiliaires 8 débouche par un orifice de sortie respectif 17 dans un évidement 14 de la paroi interne 15 de l'élément tubulaire 4. L'évidement 14 est annulaire et centré sur l'axe 16 central de révolution de l'élément tubulaire côté de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices de sortie17, ainsi qu'une face 14b suivant la face 14a et convergeant en direction de l'axe 16.

De préférence, entre la face inclinée convergente 14b et l'orifice distal 7, la paroi interne 15 présente une partie 15a légèrement évasée vers l'extérieur, comme cela est illustré par l'angle A sur la Figure 1.

Ainsi, lorsque les canaux auxiliaires 8 sont alimentés en gaz respirable(s) sous pression à travers les éléments 9 à 12, les jets gazeux correspondants heurtent la face inclinée 14b, qui les défléchit en direction de l'axe 16 (flèches F sur la Figure 1), formant ainsi des moyens de déflexion, et engendrant au voisinage de celui-ci une zone de dépression favorisant la circulation gazeuse à l'intérieur du canal principal 5, de l'orifice proximal 6 vers l'orifice distal 7. On favorise ainsi l'inspiration du patient.

On comprend qu'il est possible de réaliser les canaux auxiliaires au sein du dispositif de manière différente de celle représentée à titre d'exemple. Ainsi, par exemple, les canaux auxiliaires peuvent être des arrachements 11 ou gorges sur toutes leurs longueurs plutôt que de se poursuivre en des canaux noyés dans la paroi de l'élément tubulaire comme représentés, lesdits arrachements étant fermés extérieurement par une paroi externe tubulaire rapportée s'appliquant sur l'élément tubulaire. Ladite paroi externe tubulaire peut également former au niveau des orifices de sortie des canaux auxiliaires, les moyens de déflexion.

De préférence, la distance entre chacun des orifices de sortie 17 des canaux auxiliaires et l'orifice distal 7 est de l'ordre de 1 à 2 cm.

En aval de l'orifice distal 7, la pression dans la cavité pulmonaire est faible et pratiquement constante.

Ainsi, grâce à l'invention, on obtient une assistance respiratoire non agressive pour le patient, avec disparition quasiment totale de l'espace mort inhérent aux sondes connues.

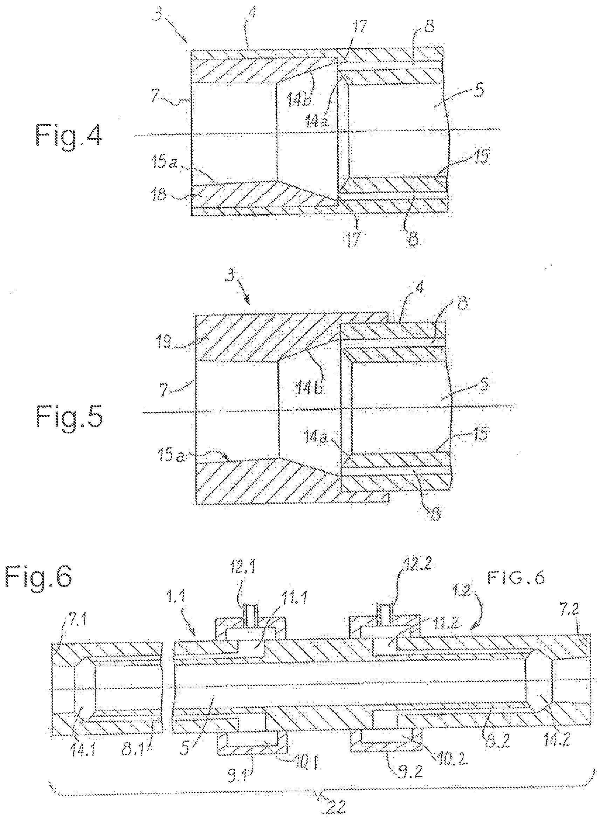

Dans le mode de réalisation de l'invention illustré par la Figure 1, on a indiqué que l'ensemble des faces 14a et 14b était réalisé par évidement de la paroi interne 15 du canal principal 5. Il va de soi que ce mode de réalisation n'est pas limitatif et que les faces 14a et 14b peuvent être obtenues de façons différentes. Par exemple, sur les Figures 4 et 5, la face 14a est formée dans la paroi interne 15 de l'élément tubulaire 4, alors que la face 14b est prévu sur un embout 18 ou 19 venant s'emboîter intérieurement, embout 18, ou extérieurement, embout 19, sur l'élément tubulaire 4.

Bien entendu, dans ce cas, l'orifice distal 7 et la paroi divergente 15a sont portés par l'embout 18 ou 19 correspondant.

Comme le montrent les Figures 2 et 3, les canaux auxiliaires 8 sont disposés régulièrement autour de l'axe de l'élément tubulaire 4. Leur nombre est variable suivant les utilisations (adulte ou enfant), mais il est généralement compris entre dix et vingt. D'une manière générale, le nombre des orifices de sortie du/des canaux auxiliaires et le diamètre des orifices de sortie et/ou des canaux auxiliaires sont ajustés afin de permettre un débit suffisant au(x) gaz respirable(s).

On remarquera qu'au moins l'un des canaux auxiliaires 8, au lieu d'être relié en commun avec les autres à la source de gaz respirable sous pression (par l'intermédiaire des éléments 9 à 12) peut être continuellement alimenté par la source de gaz respirable, de façon à maintenir une pression positive dans les poumons du patient et cela pendant ou à la fin de la phase expiratoire provoquée par l'insufflation de gaz dans les canaux auxiliaires 8 (effet anti-collapse).

Selon encore une autre variante de l'invention, un des canaux auxiliaires 8 peut également être spécialisé pour apporter un fluide médical ou un fluide d'humidification, si la source sous pression ne présente pas les caractéristiques requises.

Pour assurer l'humidification, le canal auxiliaire apportant de l'eau, de préférence tiède, est de préférence recourbé en forme de U à son extrémité distale, et vient déboucher dans une cavité ménagée dans la paroi interne 15, cavité dans laquelle débouche également un canal apportant de l'air sous pression. Dans ladite cavité, située de préférence entre l'évidement 14 et l'orifice distal 7, le canal d'eau et le canal d'air débouchent vis-à-vis l'un de l'autre, c'est-à-dire sensiblement sur le même axe, les deux fluides, air et eau, arrivant en sens contraires, ce qui permet la vaporisation de l'eau, la vapeur obtenue étant ensuite entraînée par l'air insufflé.

Au moins un canal supplémentaire 20 peut être prévu dans l'épaisseur de l'élément tubulaire 4 afin de déboucher dans la face d'extrémité distale 21 de l'élément tubulaire 4 et servir de logement à un dispositif de mesure de pression (non représenté).

Lorsqu'au moins deux prises de pression sont présentes, notamment à chacune des extrémités de l'élément tubulaire, elles permettent, grâce à la différence des pressions mesurées, de calculer le débit gazeux.

L'élément tubulaire 4 peut comporter à l'extrémité distale 3, un ballonnet gonflable (non représenté) muni des dispositifs nécessaires de sécurité ou tout autre ballonnet permettant de se comporter comme une soupape de sécurité en cas de surpression dans les poumons. Cet éventuel ballonnet peut être gonflé à partir d'un canal supplémentaire (non représenté) associé à l'élément tubulaire 4.

Un dispositif de sécurité peut être constitué simplement d'un manchon élastique entourant l'élément tubulaire, partiellement collé à ce dernier, et recouvrant une perforation pratiquée à travers la paroi dudit élément tubulaire, notamment au voisinage de l'extrémité proximale. Ainsi, lorsque la pression interne devient trop élevée, le gaz peut s'écouler à travers ladite perforation, puis entre la paroi externe de l'élément tubulaire et la paroi interne du manchon élastique. Si un manchon de sécurité est également prévu au voisinage de l'extrémité distale, la perforation correspondante, étant placée au-delà d'un éventuel ballonnet de maintien par friction, doit mettre en communication l'intérieur de l'élément tubulaire avec l'air ambiant ; ledit ballonnet doit donc être contourné, ce que l'on obtient par exemple en le plaçant autour du manchon élastique.

La variante de réalisation 22 double flux opposés du dispositif selon l'invention, montrée par la Figure 6, comporte deux dispositifs 1.1 et 1.2, chacun de structure semblable à celui de la Figure 1, inversés, accolés par leurs extrémités proximales, le dispositif 1.2 pouvant être plus court que le dispositif 1.1. Dans cette variante de réalisation 22, l'orifice distal (côté patient) du dispositif est constitué par l'orifice distal 7.1 du dispositif 1.1, tandis que l'orifice proximal (côté opposé au patient) est formé par l'orifice 7.2 du dispositif 1.2. Chacun des dispositifs 1.1 et 1.2 est pourvu de son système d'alimentation 9.1, 9.2, 10.1, 10.2, 11.1, 11.2 et 12.1, 12.2 en gaz respirable sous pression, alimentant des canaux respectifs 8.1 ou 8.2, débouchant dans des évidements annulaires 14.1 ou 14.2, proches respectivement desdits orifices 7.1 et 7.2. Le dispositif 22 forme une sonde à double flux. Ce dispositif étant en place sur le patient, c'est-à-dire le dispositif 1.1 étant relié à la voie respiratoire de celui-ci, le dispositif 1.2 et les bagues 12.1 et 12.2 étant extérieures au patient, on alimente alternativement les canaux 8.1 à travers les éléments 9.1 à 12.1 et les canaux 8.2 à travers les éléments 9.2 à 12.2 en gaz respirable(s) sous pression, de manière à favoriser alternativement l'inspiration et l'expiration du patient.

A cet effet, un dispositif (non représenté) de commutation et de réglage des débits et des durées d'insufflation de gaz est relié aux bagues 9.1 et 9.2 d'une part, et à la source de gaz d'autre part.

L'élément tubulaire respiratoire ainsi constitué permet une assistance respiratoire en insufflation et une assistance respiratoire en expiration, à condition de maintenir un débit continu assurant la sécurité et limitant le risque de collapsus pulmonaire.

La pression dans les canaux 8.2 est avantageusement plus élevée que la pression dans les canaux 8.1, car l'effet d'entraînement du fluide contenu dans la sonde n'est pas de même nature.

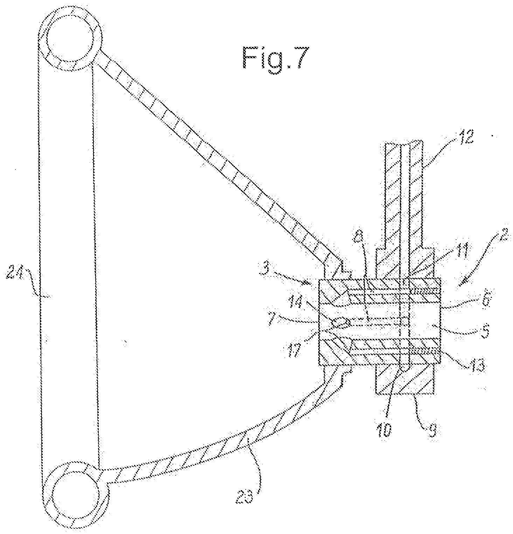

Le dispositif de la Figure 7 se distingue des dispositifs des Figures 1 à 6 d'une part par le fait que l'élément tubulaire 1 n'est pas destiné à être introduit dans ou relié à une voie respiratoire, mais constitue l'embout d'un masque et, d'autre part, par le fait que les dimensions, notamment longueur et diamètres, sont différentes. Selon la Figure 7, l'élément tubulaire principal constitue l'embout d'entrée et de sortie d'air d'un masque qui, par ailleurs, est de type connu, c'est-à-dire comprenant essentiellement une coque 23, un bourrelet d'étanchéité 24, et des moyens (non représentés) de fixation tels que des sangles.

La Figure 7 illustre aussi une variante de réalisation de l'évidement 14, les moyens de déflexion ne formant pas ici une gorge annulaire continue, mais étant constitués d'un ensemble discontinu d'évidements de forme générale conique, ménagés dans la paroi interne 15, et au fond de chacun desquels débouche par son orifice de sortie l'extrémité distale d'un canal auxiliaire 8.

Le principe de fonctionnement du dispositif de la Figure 7 est bien entendu celui décrit plus haut à propos des Figures 1 à 6.

Par ailleurs, bien que la Figure 7 comporte un élément tubulaire où l'assistance respiratoire n'agit que dans le sens de l'inspiration (comme dans le cas du dispositif de la Figure 1), un masque peut être également équipé d'un dispositif double flux opposés selon la Figure 6, pour l'assistance à l'inspiration et à l'expiration.

L'élément tubulaire 4 constitutif des modes de réalisation du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place de l'élément tubulaire, et de la taille du patient, qui peut être un adulte, un enfant, un nourrisson ou un prématuré.

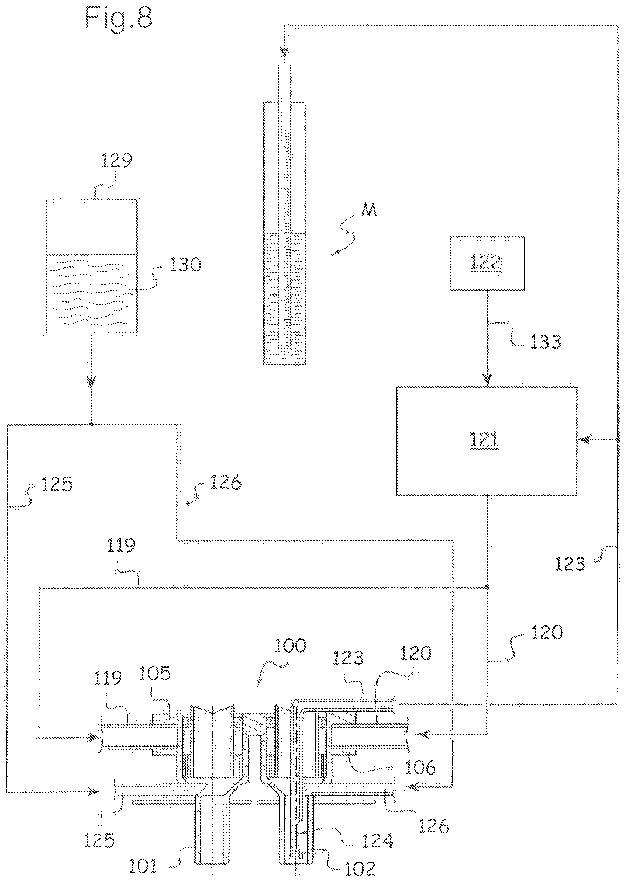

L'appareil nasal d'assistance respiratoire de la Figure 8 met en oeuvre un dispositif selon l'invention par narine, les deux dispositifs étant disposés en parallèles. La figure 8 l'illustre schématiquement avec l'appareil monté dans son circuit d'alimentation en gaz respirable(s) et en liquide d'humidification. L'appareil comporte deux dispositifs de l'invention au sein d'une pièce tubulaire monolithique 100.

Des raccords tubulaires latéraux 105 et 106 sont respectivement raccordés à des conduits d'alimentation 119 et 120, alimentés en parallèle en gaz respirable de ventilation par un dispositif d'alimentation 121, lui-même relié par une conduite 133 à une source 122 d'un tel gaz

Par ailleurs, dans le canal principal de droite sur la Figure 8, est introduit un tube capillaire 123, dont l'extrémité distale est pourvue d'une échancrure latérale 124, dont la longueur est égale à plusieurs fois le diamètre dudit tube capillaire 123. Cette échancrure latérale 124 du tube capillaire est disposée dans la partie distale du canal principal et sert de prise de pression à cet endroit. Le tube capillaire 123 transmet la pression au dispositif d'alimentation 121 et à un manomètre à eau M.

Chaque élément tubulaire est de plus associé à un conduit d'apport sous forme d'un conduit latéral 125 ou 126 débouchant dans une partie intermédiaire conique du canal principal correspondant, entre une partie de plus grand diamètre et une partie de plus petit diamètre, distale, dudit canal principal.

L'extrémité desdits conduits latéraux 125 et 126 peut faire saillie à l'intérieur du canal principal et être taillée en biseau comme représenté. L'inclinaison dudit biseau est opposée à celle de la partie intermédiaire conique.

Les conduits latéraux 125 et 126 sont reliés en parallèle à un réservoir 129 contenant un liquide 130, par exemple de l'eau, éventuellement additionnée de médicaments ou analogues, et sont alimentés en liquide à partir de ce réservoir, par exemple par gravité et/ou capillarité.

De ce qui précède, on conçoit aisément que, lorsqu'après une expiration, le patient commence à inspirer, le dispositif d'alimentation 121 reçoit la variation de pression correspondante par le tube capillaire 123 et peut adresser au patient des jets, continuels ou impulsionnels, de gaz respirable(s) par l'intermédiaire des conduits d'alimentation 119 et 120. Les jets de gaz respirable(s), formés à l'intérieur de chaque canal principal, sont défléchis par les parties intermédiaires coniques en entraînant au passage les gouttelettes du liquide 130 accrochées aux biseaux d'extrémités des conduits latéraux 125 et 126 et s'humidifient.

En revanche, lorsqu'après une inspiration, le patient commence à expirer, l'appareil d'alimentation est commandé à l'arrêt par la pression transmise par le tube capillaire 123 et le patient peut librement expirer à travers les canaux principaux.

Des rondelles de mousse (non représentées) peuvent être enfilées sur les extrémités distales 101, 102 des éléments tubulaires et servent alors de butée souple à l'enfoncement de la pièce monolithique 100 dans les narines du patient.

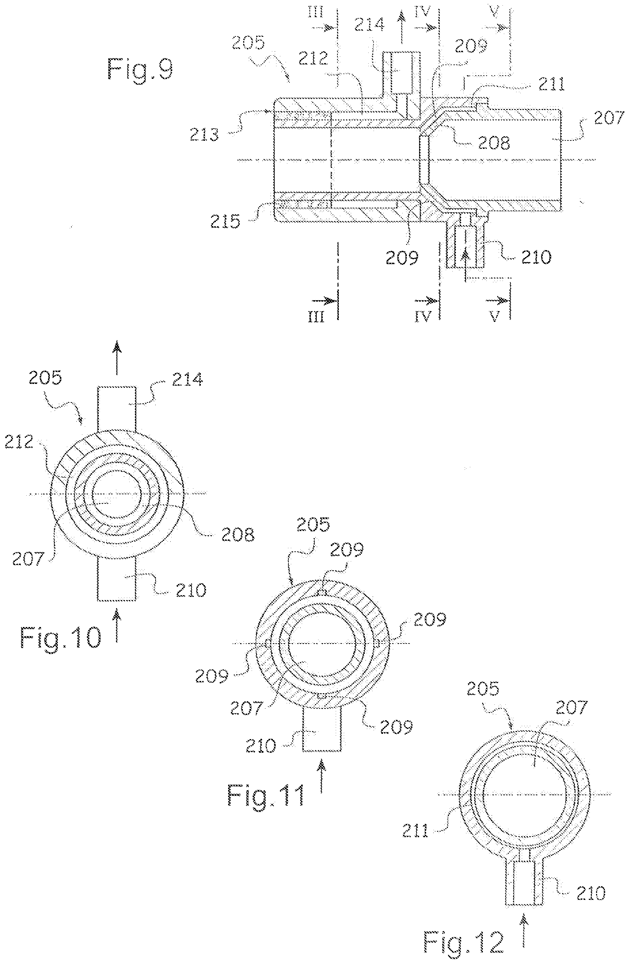

La variante 205 du dispositif, détaillée sur les figures 9 à 12, comporte un canal principal interne 207 et, en partie médiane, une paroi conique 208, saillant à l'intérieur dudit canal principal 207. La paroi conique 208 a pour objet de défléchir, en direction de l'axe du canal principal 207, des jets de gaz respirable(s) injectés par des canaux auxiliaires 209, alimentés à partir d'un embout d'amenée 210, par l'intermédiaire d'une chambre annulaire périphérique 211.

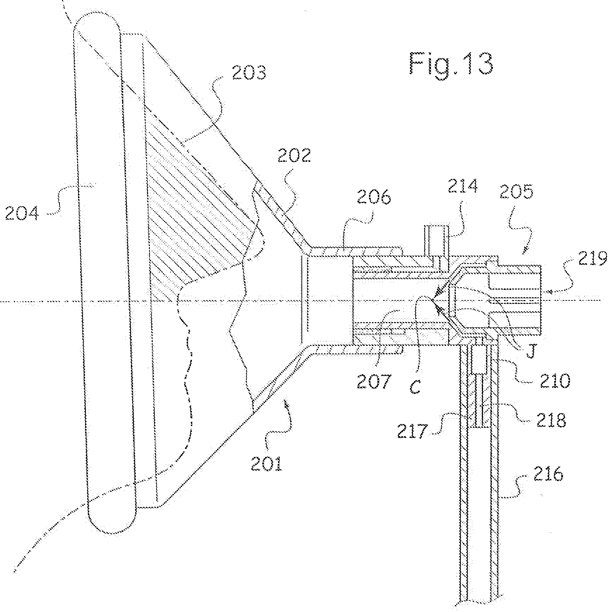

Par ailleurs, du côté distal, le dispositif 205 comporte une chambre périphérique annulaire 212, coaxiale audit dispositif 205. La chambre périphérique annulaire 212 débouche côté distal par un passage annulaire distal 213. Dans le cas où le dispositif 205 est installé sur un masque facial (représenté sur la Figure 13), la chambre périphérique annulaire 212 communique avec l'intérieur du masque par ce passage annulaire distal 213. La chambre périphérique annulaire 212 est en relation avec, côté proximal, un embout de sortie 214.

Un filtre 215, fibreux ou poreux, par exemple en coton, mousse synthétique, ou autre, est disposé dans la chambre périphérique annulaire 212, pour amortir les turbulences gazeuses et, par suite, les trop fortes variations de pression. En effet, l'embout de sortie 214 peut être relié, par exemple, à un analyseur de gaz et/ou à un dispositif de mesure de pression. Bien entendu, les liaisons entre l'embout de sortie 214 et l'analyseur de gaz et/ou à le dispositif de mesure de pression sont prévues pour que le prélèvement de gaz à analyser par la liaison n'ait pas d'influence sur l'analyse et/ou la mesure de pression, via la liaison.

Ainsi, le praticien assistant le patient connaît en permanence la composition du gaz dans le masque, notamment sa teneur en gaz carbonique, et la pression à l'intérieur dudit masque. Il peut donc prendre les mesures d'intervention appropriées en fonction desdites composition et pression du gaz.

On comprend que cette variante 205 peut être réalisée de manière différente tout en assurant les mêmes fonctions d'assistance respiratoire et de prise pour mesures. De plus, le dispositif peut également comporter en plus, des moyens ou conduits d'injection de médicaments et/ou d'eau, notamment pour nébulisation.

Plus généralement le dispositif de l'invention peut trouver de nombreuses autres applications, par exemple en combinaison avec une sonde nasale, une sonde buccale, une sonde trachéale, etc.

Le masque respiratoire 201 facial, représenté sur la figure 13, comporte une coque rigide 202 de forme générale tronconique, pouvant être appliquée sur le visage 203 d'un patient par l'intermédiaire d'un coussinet 204, bordant son ouverture périphérique. Du côté opposé au visage, le masque 201 est pourvu d'un dispositif d'assistance respiratoire conforme à la présente invention, comportant un élément tubulaire 205, solidaire de ladite coque 202 ou emboîté sur une saillie tubulaire 206 de celle-ci. L'élément tubulaire 205 sert d'embout d'entrée et de sortie de gaz dans le masque 201, son extrémité proximale, opposée au patient, étant à l'air libre, alors que son extrémité distale, côté patient, se raccorde au masque 201. Comme précédemment, l'élément tubulaire 205 forme un canal principal 207 interne et il comporte, en partie médiane, des moyens de déflexion, dirigés vers l'axe central dudit canal principal. Les moyens de déflexion ont pour objet de défléchir, en direction de l'axe central du canal principal, des jets de gaz respiratoire J injectés par des orifices de sortie aux extrémités de canaux auxiliaires périphériques, alimentés à partir d'un embout d'amenée 210, par l'intermédiaire d'une chambre annulaire périphérique, lesdits jets de gaz respiratoire convergeant ainsi vers un point de convergence C de l'axe central dudit canal principal 207. Le gaz respiratoire est amené à la chambre annulaire périphérique et aux canaux auxiliaires périphériques par un conduit d'alimentation 216 relié, d'un côté à l'embout d'amenée 210 et, de l'autre, à une source de gaz respiratoire(s), telle qu'une bouteille de gaz sous pression (non représentée). A l'intérieur du conduit d'alimentation 216 est disposé un bouchon 217, percé d'un passage longitudinal 218. Par exemple, le bouchon 217 peut être obtenu par découpe d'un tronçon sur la longueur d'un profilé dont le passage longitudinal présente un diamètre d constant. On comprendra aisément qu'en faisant varier la longueur du bouchon et de son tronçon, on fait varier la perte de charge apportée par ce dernier dans le conduit d'alimentation 216. On peut ainsi, par expérimentation, régler ladite perte de charge pour que, en utilisation, la pression du gaz respiratoire à l'extrémité distale de l'élément tubulaire 205 corresponde à une oxygénation optimale et sans danger, du patient. Par ailleurs, l'élément tubulaire 205 comporte un embout 214 pour le prélèvement de gaz et/ou la mesure de pression. Dans ce mode de réalisation, on a prévu dans l'extrémité proximale de l'élément tubulaire 205 des obstacles, par exemple des ailettes convergentes 219, empêchant l'introduction accidentelle d'un objet pouvant obturer hermétiquement ladite extrémité proximale.

Comme on l'a vu, le dispositif de l'invention peut être utilisé combiné avec un masque facial. Cependant, le dispositif de l'invention peut, plus généralement, être utilisé en combinaison avec tout autre appareil utile à l'assistance respiratoire, comme par exemple un masque laryngé, et aussi en toute position notamment supraglottique ou supratrachéale.

Le dispositif de l'invention peut être utilisé dans les tentatives de réanimation de personnes en arrêt respiratoire et/ou cardiaque. Ces personnes peuvent produire des rejets liquides de type expectorations, hémorragies, hémoptysies, voire vomissements lorsqu'elles ne sont pas encore intubées, etc. qui peuvent passer dans les voies respiratoires et être expulsés à travers le dispositif avec le risque de les disperser par la sortie/extrémité proximale et de provoquer des souillures possiblement contaminantes. On prévoit donc, dans une variante, des moyens de filtrage/retenue de ces rejets liquides côté extrémité proximale du dispositif. Ces moyens peuvent être un filtre, par exemple un adaptateur dans lequel une masse de filtrage est installée ou peut l'être, ladite masse pouvant par exemple être une compresse dont la texture permet le passage des gaz. Ces moyens peuvent être un décanteur avec un récipient de décantation, de préférence à distance du dispositif et relié à ce dernier par un tube souple. Dans le cas où le dispositif comporte en outre des moyens fonctionnels additionnels côté proximal, par exemple des moyens de freinage, alors, de préférence, les moyens de filtrage/retenue sont disposés entre le dispositif et ces moyens fonctionnels additionnels. Dans une variante plus simple, un tube est simplement relié à l'extrémité proximale du dispositif par sa première extrémité et l'autre extrémité du tube est positionnée à un emplacement où d'éventuelles souillures sont sans importance et/ou récoltées.

Enfin, dans une variante de mise en oeuvre présentée dans la demande WO2008113913 et qui peut être employée dans le cadre de la présente invention, le dispositif comporte en outre des moyens de freinage spontané ou commandé de l'entrée d'air extérieur dans le canal principal via son extrémité proximale. Ces moyens peuvent être installés à demeure sur le dispositif et faire partie intégrante de celui-ci ou, alors, être amovibles par raccordement sur l'extrémité proximale dudit dispositif. Grâce à ces moyens de freinage, il est possible d'utiliser le dispositif pour la réanimation d'une personne en état d'arrêt cardiaque. Dans ce cas, on utilise le dispositif à moyens de freinage pour la ventilation, par exemple au masque ou par intubation, et on exerce des compressions et des décompressions alternées sur la cage thoracique de ladite personne. Il se produit alors un freinage de l'entrée de l'air extérieur dans le dispositif et donc vers le patient, au début de chaque décompression. Il en résulte une baisse de pression pulmonaire dans la cage thoracique et donc sur le coeur et donc une aide à l'expansion cardiaque et au retour sanguin vers le coeur. Le freinage proximal spontané est essentiellement passif et met en oeuvre des soupapes tarées qui s'ouvrent ou se ferment en fonction d'une différence de pression. Le freinage proximal commandé est essentiellement actif et un moyen de restriction commandé du canal principal est mis en oeuvre. Ce moyen de restriction est commandé en fonction de mesures d'un capteur détectant au moins un début de décompression/relâchement du massage thoracique. Dans des variantes, on peut combiner ce freinage proximal passif ou actif avec une modulation des jets des canaux auxiliaires en fonction des cycles inspiratoires et expiratoires, voire même remplacer le freinage proximal par cette modulation des jets, car une augmentation de la vitesse des jets revient à augmenter l'effet de soupape distale et à créer/augmenter le freinage en distal du passage des gaz du canal principal, et inversement si on réduit les jets. On comprend que ce freinage distal par les jets des canaux auxiliaires est actif et, en pratique, on augmente la pression et débit dans les canaux auxiliaires lors d'un début d'inspiration détecté par un capteur.

## Revendications

1. Dispositif d'assistance respiratoire (1) pour un patient comportant un élément tubulaire formant un canal principal (5) (207) qui est destiné à être relié par son extrémité distale (3) à une voie respiratoire du patient, ledit canal principal reliant à l'extérieur le système respiratoire dudit patient par son extrémité proximale (2), ledit dispositif comportant en outre au moins un canal auxiliaire (8) (209) permettant l'injection par des orifices de sortie distaux (17) du/des canaux auxiliaires de jets de gaz respirable(s) destiné(s) à la ventilation dudit patient, lesdits orifices de sortie débouchant dans ledit canal principal au voisinage de l'extrémité distale (3) de ce dernier, le diamètre de chacun des orifices de sortie distaux étant inférieur à 150 microns, des moyens de déflexion (14b) (14a) permettant la déflexion des jets de gaz respirable(s) vers l'intérieur dudit canal principal,
**caractérisé en ce qu'**il comporte au moins un orifice de sortie en forme de bande annulaire ou semi-annulaire résultant de la réunion d'au moins certains des orifices de sortie distaux, l'épaisseur de chaque orifice de sortie en forme de bande étant inférieure à 150 microns.

2. Dispositif selon la revendication 1, **caractérisé en ce que** l'épaisseur de chaque orifice de sortie en bande annulaire ou semi-annulaire est comprise entre 50 microns et 10 microns et est, de préférence d'environ 25 microns.

3. Dispositif selon la revendication 1 ou 2, **caractérisé en ce qu'**il comporte, en outre du/des orifices de sortie en bandes semi-annulaires, des orifices de sortie ponctuels, lesdits orifices ponctuels ayant un diamètre inférieur à 150 microns.

4. Dispositif selon la revendication 1, 2 ou 3, **caractérisé en ce que** les orifices de sortie en bande(s) résultent de la réunion d'au moins certains des orifices de sortie distaux et de leurs canaux auxiliaires correspondants.

5. Dispositif selon la revendication 1, 2 ou 3, **caractérisé en ce qu'**il comporte un ensemble de canaux auxiliaires (8) (209), lesdits canaux auxiliaires étant sensiblement parallèles à l'axe central dudit élément tubulaire sur au moins une partie de leurs trajets.

6. Dispositif selon la revendication 4, **caractérisé en ce qu'**il comporte un seul canal auxiliaire commun sur lequel les orifices de sortie (17) sont reliés, ledit canal auxiliaire commun étant un passage annulaire externe sensiblement coaxial et parallèle à l'axe central dudit élément tubulaire.

7. Dispositif selon l'une quelconque des revendications précédentes, **caractérisé en ce qu'**il comporte au moins une couronne d'orifices de sortie (17), les orifices de sortie d'une couronne étant disposés le long d'une section transversale dudit élément tubulaire.

8. Dispositif selon l'une quelconque des revendications précédentes, **caractérisé en ce que** les moyens de déflexion (14b) (14a) sont configurés de manière à permettre d'orienter les jets vers l'extrémité distale de l'élément tubulaire selon une incidence par rapport à l'axe central (16) dudit élément tubulaire comprise entre 90°, l'axe des jets étant perpendiculaire audit axe central, et 25°, l'axe des jets croisant l'axe central selon un angle de 25°.

9. Dispositif selon l'une quelconque des revendications précédentes, **caractérisé en ce qu'**il est à double flux inversés permettant de favoriser l'expiration aussi bien que l'inspiration d'un patient, le dispositif comportant au moins un canal auxiliaire supplémentaire (8.2), indépendant du/des premiers canaux auxiliaires (8.1) des jets de l'extrémité distale du canal principal, et relié à une source de gaz sous pression, ledit au moins un canal auxiliaire supplémentaire débouchant dans le canal principal au voisinage de l'extrémité proximale de ce dernier, et au moins l'/les extrémités proximales dudit/desdits canaux auxiliaires supplémentaires débouchant par des orifices de sortie proximaux respectifs dans le canal principal est/sont parallèle à celui-ci, tandis que, en regard de chaque orifice de sortie proximal du canal auxiliaire supplémentaire correspondant, sont prévus des moyens pour la déflexion du jet de gaz traversant ce dernier en direction de l'intérieur dudit canal principal.

10. Dispositif selon l'une quelconque des revendications précédentes, **caractérisé en ce qu'**il comporte en outre au moins un conduit d'apport (125, 126) alimenté en produit liquide à nébuliser dans le canal principal et des moyens de nébulisation.

11. Dispositif selon l'une quelconque des revendications précédentes, **caractérisé en ce qu'**il comporte en outre au moins une prise de pression disposée du côté de l'extrémité distale de l'élément tubulaire.

12. Dispositif selon la revendication 11, **caractérisé en ce que** la prise de pression comporte une chambre périphérique annulaire (212) coaxiale à l'élément tubulaire et débouchant côté distal du dispositif par un passage annulaire distal (213), ladite chambre périphérique annulaire étant en relation avec un embout de sortie (214) latéral, un filtre (215), fibreux ou poreux annulaire étant disposé dans la chambre périphérique annulaire.

13. Dispositif selon l'une quelconque des revendications précédentes, **caractérisé en ce qu'**il est spécialement configuré pour être mis en oeuvre sur un patient en arrêt cardiorespiratoire et sur lequel un massage thoracique est effectué avec des phases de compression thoracique pour expiration et de relâchement thoracique pour inspiration et **en ce qu'**il comporte en outre dans son canal principal au moins un moyen de freinage proximal de la circulation d'air et/ou d'oxygène inspiré par l'extrémité proximale en début d'inspiration afin d'augmenter la dépression intrathoracique au début de l'inspiration, ledit moyen de freinage étant soit un moyen passif, soit un moyen actif, le moyen passif étant choisi parmi une/des soupapes à membrane souple et/ou une/des soupapes à clapets, le moyen actif comportant un équipement de mesure de pression distale, un équipement de commande et un équipement commandé de réduction de passage d'une partie proximale du canal principal, l'équipement de commande étant configuré pour commander la réduction de passage par l'équipement commandé seulement lors du début d'une inspiration détectée par des mesures de l'équipement de mesure de pression distale.

14. Appareil nasal d'assistance respiratoire (100) pour un patient, **caractérisé en ce qu'**il comporte, pour chaque narine du patient un dispositif selon l'une quelconque des revendications précédentes, les deux dispositifs étant destinés à être alimentés en gaz respirable(s) en parallèle, chaque canal principal d'un dispositif étant destiné à être relié par son extrémité distale au système respiratoire du patient par l'intermédiaire d'une de ses narines.

15. Masque d'assistance respiratoire (23) (201), **caractérisé en ce qu'**il est muni d'un dispositif selon l'une quelconque des revendications 1 à 13.

## Patentansprüche

1. Atemhilfevorrichtung (1) für einen Patienten, mit einem schlauchartigen Element, das einen Hauptkanal (5) (207) bildet, der dazu bestimmt ist, durch sein distales Ende (3) mit einem Luftweg des Patienten verbunden zu werden, wobei der Hauptkanal das Atmungssystem des Patienten durch sein proximales Ende (2) mit der Umgebung verbindet, wobei die Vorrichtung außerdem wenigstens einen Hilfskanal (8) (209) aufweist, der durch distale Ausgangslöcher (17) des Hilfskanals (der Hilfskanäle) ein Einspritzen von Strahlen von für die Beatmung des Patienten bestimmtem (bestimmen) Beatmungsgas(en) ermöglicht, wobei die Ausgangslöcher in der Nähe des distalen Endes (3) des Hauptkanals in selbigen münden, wobei der Durchmesser jedes der distalen Ausgangslöcher kleiner als 150 Mikron ist, wobei Ablenkmittel (14b) (14a) ein Ablenken der Strahlen des Beatmungsgases (der Beatmungsgas) zum Inneren des Hauptkanals hin ermöglichten, **dadurch gekennzeichnet, daß** sie wenigstens ein Ausgangsloch in Form eines ringförmigen oder halbringförmigen Bands aufweist, das aus der Vereinigung wenigstens einiger der distalen Ausgangslöcher herrührt, wobei die Dicke jedes Ausgangslochs in Form eines Bands kleiner als 150 Mikron ist.

2. Vorrichtung gemäß Anspruch 1, **dadurch gekennzeichnet, daß** die Dicke jedes Ausgangslochs in Form eines ringförmigen oder halbringförmigen Bands zwischen 50 Mikron und 10 Mikron und vorzugsweise etwa 25 Mikron beträgt.

3. Vorrichtung gemäß Anspruch 1 oder 2, **dadurch gekennzeichnet, daß** sie außer den Ausgangslöchern in Form eines halbringförmigen Bands einzelne Ausgangslöcher aufweist, wobei die einzelnen Ausgangslöcher einen Durchmesser von weniger als 150 Mikron aufweisen.

4. Vorrichtung gemäß Anspruch 1, 2 oder 3, **dadurch gekennzeichnet, daß** die bandförmigen Ausgangslöcher aus der Vereinigung wenigstens einiger der distalen Ausgangslöcher und deren entsprechenden Hilfskanälen herrühren.

5. Vorrichtung gemäß Anspruch 1, 2 oder 3, **dadurch gekennzeichnet, daß** sie eine Gesamtheit von Hilfskanälen (8) (209) aufweist, wobei die Hilfskanäle wenigstens über einen Teil von deren Verlauf im Wesentlichen parallel zur Mittelachse des schlauchförmigen Elements sind.

6. Vorrichtung gemäß Anspruch 4, **dadurch gekennzeichnet, daß** sie einen einzigen gemeinsamen Hilfskanal aufweist, mit dem die Ausgangslöcher (17) verbunden sind, wobei der gemeinsame Hilfskanal ein äußerer ringförmiger, im Wesentlichen koaxialer und zur Mittelachse des schlauchförmigen Elements paralleler Durchgang ist.

7. Vorrichtung gemäß einem der vorangehenden Ansprüche, **dadurch gekennzeichnet, daß** sie wenigstens einen Kranz von Ausgangslöchern (17) aufweist, wobei die Ausgangslöcher eines Kranzes entlang eines Querschnitts des schlauchförmigen Elements angeordnet sind.

8. Vorrichtung gemäß einem der vorangehenden Ansprüche, **dadurch gekennzeichnet, daß** die Ablenkmittel (14b) (14a) so ausgestaltet sind, daß sie ermöglichen, die Strahlen zum distalen Ende des schlauchförmigen Elements hin unter einer Neigung gegenüber der Mittelachse (16) auszurichten, die zwischen 90°, wobei dann die Strahlen zur Mittelachse senkrecht sind, und 25°, wobei dann die Achse der Strahlen die Mittelachse unter einem Winkel von 25° schneidet, beträgt.

9. Vorrichtung gemäß einem der vorangehenden Ansprüche, **dadurch gekennzeichnet, daß** sie für Umkehr-Doppelfluß ausgelegt ist, was ermöglicht, sowohl das Ausatmen als auch das Einatmen des Patienten zu fördern, wobei die Vorrichtung mindestens einen weiteren Hilfskanal (8.2) aufweist, der vom Hilfskanal (den Hilfskanälen) der Strahlen des distalen Endes des Hauptkanals unabhängig ist und mit einer Druckgasquelle verbunden ist, wobei der wenigstens eine zusätzliche Hilfskanal in der Nähe des proximalen Endes des Hauptkanals in letzteren mündet und wobei das (die) proximale(n) Ende(n) des oder der zusätzlichen Hilfskanäle, die durch entsprechende proximale Ausgangslöcher in den Hauptkanal münden, zu diesem parallel ist (sind), während gegenüber jedes proximalen Ausgangslochs des entsprechenden zusätzlichen Hilfsltanals Mittel zum Ablenken des Gasstrahls vorgesehen sind, die letzteren mit Richtung zum Inneren des Hauptkanals hin kreuzen.

10. Vorrichtung gemäß einem der vorangehenden Ansprüche, **dadurch gekennzeichnet, daß** sie außerdem wenigstens eine Zuführleitung (125, 126), die mit einem flüssigen, im Hauptkanal zu versprühenden Produkt versorgt wird, und Versprühmittel aufweist.

11. Vorrichtung gemäß einem der vorangehenden Ansprüche, **dadurch gekennzeichnet, daß** sie außerdem wenigstens einen auf der Seite des distalen Endes des schlauchförmigen Elements angeordneten Druckanschluß aufweist.

12. Vorrichtung gemäß Anspruch 11, **dadurch gekennzeichnet, daß** der Druckanschluß eine ringförmige, zum schlauchförmigen Element koaxiale und am distalen Ende der Vorrichtung durch einen distalen ringförmigen Durchgang (213) mündende Umfangskammer (212) aufweist, wobei die ringförmigen Umfangskammer mit einem seitlichen Ausgangsstück (214) in Verbindung steht, wobei ein faseriger oder poröser ringsförmiger Filter (215) in der ringförmigen Umfangskammer angeordnet ist.

13. Vorrichtung gemäß einem der vorangehenden Ansprüche, **dadurch gekennzeichnet, daß** sie außerdem speziell dazu ausgelegt ist, bei einem Patienten angewendet zu werden, der einen Herz-Kreislauf-Stillstand erlitten hat und bei dem eine Brustkorbmassage mit Phasen der Kompression des Brustkorbs zum Ausatmen und des Loslassens des Brustkorbs zum Einatmen durchgeführt wird, und daß sie außerdem in ihrem Hauptkanal wenigstens ein proximales Mittel zum Bremsen des Flusses der durch das proximale Ende am Anfang des Einatmens eingeatmeten Luft und/oder des Sauerstoffs, um den inneren Unterdruck im Brustkorb am Anfang des Einatmens zu erhöhen, wobei das Bremsmittel entweder ein passives oder ein aktives Mittel ist, wobei das passive Mittel unter einem oder mehreren Ventilen mit flexibler Membrane und/oder einem oder mehreren Klappenventile ausgewählt wird, wobei das aktive Mittel eine distale Druckmeßausrüstung, eine Steuerungsausrüstung und eine gesteuerte Ausrüstung zum Reduzieren des Durchflusses eines proximalen Teils des Hauptkanals aufweist, wobei die Steuerungsausrüstung dazu ausgelegt ist, das Reduzieren des Durchflusses durch die gesteuerte Ausrüstung nur am Anfang eines durch Messungen der distalen Druckmeßausrüstung festgestellten Einatmens zu steuern.

14. Nasale Atemhilfevorrichtung (100) für einen Patienten, **dadurch gekennzeichnet, daß** sie für jedes Nasenloch des Patienten eine Vorrichtung gemäß einem der vorangehenden Ansprüche aufweist, wobei die beiden Vorrichtungen dazu bestimmt sind, parallel mit Beatmungsgas(en) versorgt zu werden, wobei jeder Hauptkanal einer Vorrichtung dazu bestimmt ist, durch sein distales Ende über eins der Nasenlöcher des Patienten mit dessen Atmungssystem verbunden zu sein.

15. Atemhilfemaske (23) (201), **dadurch gekennzeichnet, daß** sie mit einer Vorrichtung gemäß einem der Ansprüche 1 bis 13 ausgestattet ist.

## Claims

1. Device (1) for respiratory assistance of a patient comprising a tubular element forming a main channel (5) (207) intended for being connected by its distal end (3) to a respiratory tract of the patient, said main channel connecting the patient's respiratory system by its proximal end (2) to the outside, said device further comprising at least one auxiliary channel (8) (209) allowing for injection of a jet or of jets of respirable gas through distal outlet holes (17) of the auxiliary channel or channels and intended for ventilation of said patient, said outlet hole opening-out in the main channel in the vicinity of the distal end (3) of the latter, the diameter of each of the distal outlet holes being less than 150 micrometres, deflecting means (14b) (14a) allowing to deflect the jets of respirable gas toward the interior of said main channel,
**characterised in that** it comprises art least one outlet hole shaped as an annular or semi annular strip resulting from uniting together at least some of the distal outlet holes, the thickness of the outlet hole shaped as a strip being less than 150 micrometres.

2. Device according to claim 1, **characterised in that** the thickness of each outlet hole shaped as an annular or semi annular strip is comprised between 50 micrometres and 10 micrometres and is preferably about 25 micrometres.

3. Device according to claim 1 or 2, **characterised in that** it comprises, in addition to the outlet holes shaped as annular or semi annular strips, individual outlet holes, said individual holes having a diameter of less than 150 micrometres.

4. Device according to claim 1, 2 or 3, **characterised in that** the outlet holes shaped as a strip or as strips result from uniting together at least certain ones of the distal outlet holes and of their corresponding auxiliary channels.

5. Device according to claim 1, 2 or 3, **characterised in that** it comprises a group of auxiliary channels (8) (209), said auxiliary channels being approximately parallel to the central axis of said tubular element on at least part of their way.

6. Device according to claim 4, **characterised in that** it comprises a single common auxiliary channel to which the outlet holes (17) are connected, said common auxiliary channel being an external annular passage way approximately coaxial and parallel to the central axis of said tubular element.

7. Device according to any of the preceding claims, **characterised in that** it comprises at least one crown of outlet holes (17), the outlet holes of one crown being located along a transverse section of said tubular element.

8. Device according to any of the preceding claims, **characterised in that** the deflection means (14b) (14a) are arranged as to allow the jets to be oriented toward the distal end of the tubular element with an inclination with respect to the central axis (16) of said tubular element which is between 90°, the axis of the jets then being perpendicular to said central axis, and 25°, the axis of the jets then crossing the central axis with an angle of 25°.

9. Device according to any of the preceding claims, **characterised in that** it is arranged as a two-inverted-flows device, allowing for favouring both exhaling and inhaling of a patient, the device comprising at least one supplemental auxiliary channel (8.2), independent from the first auxiliary channel or channels (8.1) of the jets of the distal end of the main channel and connected to a source of pressurized gas, said at least one supplemental channel opening-out in the main channel in the vicinity of the proximal end of the latter, and at least the proximal end or ends of said supplemental auxiliary channel or channels which open-out with corresponding proximal outlet holes in the main channel, is or are parallel to it, whereas in front of each proximal outlet hole of the corresponding supplemental auxiliary channel, means are provided for deflecting the gas jet passing through the latter toward the interior of said main channel.

10. Device according to any of the preceding claims, **characterised in that** it further comprises at least one feeding tube (125, 126), supplied with a liquid substance for being nebulized into the main channel, and nebulization means.

11. Device according to any of the preceding claims, **characterised in that** it further comprises at least one pressure tap located at the side of the distal end of the tubular element.

12. Device according to claim 11, **characterised in that** the pressure tap comprises an annular peripheral chamber (212) which is coaxial to the tubular element and ends at the distal side of the device by an annular distal passage way (213), said annular peripheral chamber being in relation to a lateral outlet nipple (214), an annular fibrous or porous filter (215) being located in the annular peripheral chamber.

13. Device according to any of the preceding claims, **characterised in that** it is specifically arranged for being used for a patient suffering from cardiac and respiratory arrest and to whom a massage of the thoracic cage is applied with phases of compression of the thoracic cage for exhaling and release phases for inhaling, and **in that** it further comprises in its main channel at least one means for proximally slowing down the flow of air and/or oxygen inhaled through the proximal end at the beginning of inhaling in order to increase the depression inside the thoracic cage at the beginning of inhaling, said slowing down means being either a passive means or an active means, the passive means being chosen from one or more valves having a flexible membrane and/or one or more valves having a flap, the active means comprising an equipment for measuring the distal pressure, a control equipment, and a controlled equipment for reducing the passage way of a proximal part of the main channel, the control equipment being arranged for controlling the reduction of the passage way by the controlled means only at the beginning of inhaling detected by the measurements obtained from the equipment for measuring the distal pressure.

14. Nasal apparatus (100) for respiratory assistance for a patient, **characterised in that** it comprises for each nostril of the patient a device according to any one of the preceding claims, both devices being intended to be supplied in parallel with respirable gas or gases, each main channel of a device being intended for being connected by its distal end to the respiratory system of the patient by one of his nostrils.

15. Mask (23) (201) for respiratory assistance, **characterised in that** it comprises a device according to any of claims 1 to 13.
